(19) Europäisches Patentamt
European Patent Office
Office européen des brevets

(11) Veröffentlichungsnummer: **0 068 372**
**B1**

(12) **EUROPÄISCHE PATENTSCHRIFT**

(45) Veröffentlichungstag der Patentschrift: **05.12.84**

(51) Int. Cl.³: **C 07 C 69/16,** C 07 C 67/29

(21) Anmeldenummer: **82105400.4**

(22) Anmeldetag: **19.06.82**

(54) **Verfahren zur Herstellung von 2-Alkyl-4,4-diacyloxy-2-butenalen.**

(30) Priorität: **01.07.81 DE 3125891**

(43) Veröffentlichungstag der Anmeldung: **05.01.83 Patentblatt 83/1**

(45) Bekanntmachung des Hinweises auf die Patenterteilung: **05.12.84 Patentblatt 84/49**

(84) Benannte Vertragsstaaten: **BE CH DE FR GB IT LI**

(56) Entgegenhaltungen: **EP - A - 0 005 452**

(73) Patentinhaber: **BASF Aktiengesellschaft, Carl-Bosch-Strasse 38, D-6700 Ludwigshafen (DE)**

(72) Erfinder: **Fischer, Rolf, Dr., Bergstrasse 98, D-6900 Heidelberg (DE)**
Erfinder: **Weitz, Hans-Martin, Dr., Auf dem Koeppel 40, D-6702 Duerkheim (DE)**
Erfinder: **Paust, Joachim, Dr., Ringstrasse 3, D-6708 Neuhofen (DE)**

**EP 0 068 372 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von 2-Alkyl-4,4-diacyloxy-2-butenalen (2-Alkylfumardialdehyd-4-monoacylalen) durch Umsetzung von 2-Alkyl-1,4-diacyloxy-1,3-butadienen mit Sauerstoff oder Sauerstoff übertragenden Verbindungen in Gegenwart von Carbonsäuren.

2-Alkylfumardialdehyd-4-monoacetale und 4-Monoacylale sind wertvolle Bausteine für die Synthese verschiedener Terpene mit biologischer und pharmakologischer Wirksamkeit. Für ihre Herstellung sind schon mehrere Verfahren vorgeschlagen worden. So ist aus den DE-OS Nrn. 2357752 und 2357810 bekannt, dass sich 3-Methyl-2-buten-1-alacetale und entsprechende Acylale mit Selendioxid zu Trans-2-methyl-2-buten-1,4-dial-4-acetalen und entsprechenden Acylalen oxidieren lassen. Nach dem in der DE-OS Nr. 2225612 beschriebenen Verfahren lassen sich cyclische 3-Methyl-2-buten-4-ol-1-alacetale mit schwefelsaurer Chromsäurelösung zu entsprechenden 2-Methyl-2-buten-1,4-dial-4-monoacetalen oxidieren.

Trans-2-methyl-2-buten-1,4-dial-4-acetale können auch nach den Angaben der DE-OS Nr. 2513999 in einem vielstufigen Verfahren hergestellt werden, wobei man in einem ersten Schritt Krotonaldehydacetale einer Ozonolyse unterwirft. In der EP-PS Nr. 9752 wird die Umsetzung von Sechsringacetalen des 3-Methyl-3-buten-1-als mit Nitrosierungsmitteln wie Nitrosylchlorid oder Salpetrigsäureestern in Gegenwart von Methanol und Salzsäure beschrieben. Aus den dabei gebildeten 2-Chlor-2-methylbutan-1,4-dialbisacetalen spaltet man mit Basen Chlorwasserstoff ab. Die so erhaltenen 2-Methyl-2-buten-1,4-dialbisacetale lassen sich mit verdünnten wässerigen Säuren selektiv zu 2-Methylfumardialdehyd-4-monoacetalen verseifen.

Es wurde nun gefunden, dass man 2-Alkyl-4,4-diacyloxy-2-butenale der Formel:

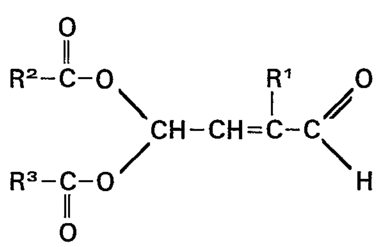
(I)

in der $R^1$ einen Alkylrest mit 1 bis 5 Kohlenstoffatomen und $R^2$ und $R^3$ ein Wasserstoffatom, einen aliphatischen Rest mit 1 bis 15 Kohlenstoffatomen, einen cycloaliphatischen Rest mit 5 bis 7 Kohlenstoffatomen oder einen aromatischen Rest bedeuten, besonders vorteilhaft dadurch herstellen kann, dass man 2-Alkyl-1,4-diacyloxy-1,3-butadiene der Formel:

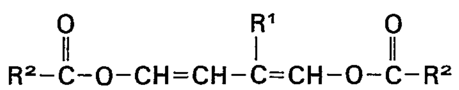
(II)

in der $R^1$ und $R^2$ die obengenannte Bedeutung besitzen, mit Sauerstoff oder Sauerstoff übertragenden Verbindungen und in Gegenwart von Carbonsäuren der Formel:

$$R^3-COOH \qquad (III)$$

in der $R^3$ die obengenannte Bedeutung hat, umsetzt.

Nach dem neuen Verfahren werden die 2-Alkyl-4,4-diacyloxy-2-butenale aus den 2-Alkyl-1,4-diacyloxy-1,3-butadienen in einem technisch einfach durchführbaren Reaktionsschritt mit hoher Selektivität und überwiegend als Transisomere erhalten.

Die Reaktion lässt sich für den Fall der Herstellung von 2-Methyl-4,4-diacetoxy-2-butenalformal durch die folgenden Formeln anschaulich machen

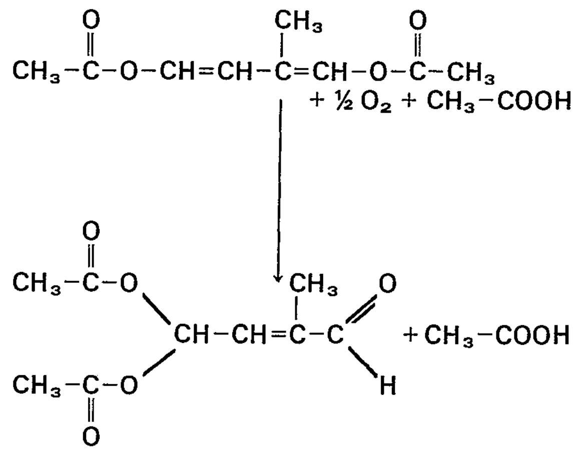


Die bei dieser Reaktion durchlaufenen Zwischenverbindungen brauchen nicht isoliert zu werden.

Die als Ausgangsstoffe verwendeten 2-Alkyl-1,4-diacyloxy-1,3-butadiene der Formel II enthalten als Rest $R^1$ einen Alkylrest mit 1 bis 5, vorzugsweise 1 bis 3 C-Atomen. Die beiden Reste $R^2$, die gleich oder verschieden sein können, bedeuten Wasserstoffatome, aliphatische Reste mit 1 bis 15 C-Atomen, cycloaliphatische Reste mit 5 bis 7 C-Atomen oder aromatische Reste. Aliphatische Reste sind z.B. Alkylreste, wie Methyl-, Ethyl-, n-Propyl-, i-Propyl-, n-Butyl-, i-Butyl-, tert.-Butyl-, n-Pentyl-, Palmityl- oder Stearylreste.

Als cycloaliphatische Reste kommen z.B. Cyclopentyl-, Cyclohexyl- oder Cycloheptylreste in Betracht. Aromatische Reste können z.B. Phenylreste sein, die gegebenenfalls durch Alkyl- oder Halogenreste substituiert sein können.

Beispielsweise seien die folgenden Verbindungen der Formel II genannt: 2-Methyl-, 2-Ethyl-, 2-n-Propyl-, 2-Butyl-, 2-Pentyl-1,4-diacetoxy-1,3-butadien, 2-Methyl-1-propionyloxy-4-acetoxy-1,3-butadien, 2-Methyl-1-acetoxy-4-palmityloxy-1,3-butadien, 2-Methyl-1-cyclohexyloxy-4-acetoxy-1,3-butadien, 2-Methyl-1-benzoyloxy-4-acetoxy-1,3-butadien.

Die Ausgangsprodukte der Formel II lassen sich z.B. durch Acetylierung von 2-Alkyl-4-acyloxy-2-butenalen mit Acetanhydrid („J. Org. Chem.", *41*, 2625 [1976]) oder durch Pyrolyse von 2-Alkyl-3,4-diacetoxytricyclo-[4,2,1,$0^{2,5}$]-non-7-enen („Journal of Chemical Society, Chemical Communications", *1974*, S. 956 bis 957) herstellen.

In den Carbonsäuren der Formel III hat der Rest $R^3$ die oben für den Rest $R^2$ genannte Bedeutung, wobei der Rest $R^3$ jedoch jeweils von den Resten $R^2$ verschieden sein kann. Als Carbonsäuren der Formel III kommen z.B. Ameisen-, Essig-, Propion-, Butter-, Valerian-, Caprin-, Laurin-, Öl-, Palmitin-, Cyclohexancarbon-, Benzoe- oder Phenylessigsäure in Betracht. Essigsäure ist aus wirtschaftlichen Gründen besonders bevorzugt. Die Carbonsäure wird im allgemeinen im Überschuss, z.B. in der 1 bis 80 molaren Menge, bezogen auf das eingesetzte 1,3-Dien II angewandt.

Sauerstoff kann in Form von reinem Sauerstoff oder im Gemisch mit anderen Gasen wie Stickstoff z.B. in Form von Luft oder mit anderen inerten Gasen wie Kohlendioxid verwendet werden.

Als Sauerstoff übertragende Verbindungen sind z.B. solche geeignet, die für die Epoxidierung von Olefinen benutzt werden, wie Wasserstoffperoxid, Per-, Perameisen-, Peressig-, Perpropion-, Perbenzoe-, Per-n-butter-, Perisobuttersäure und organische Hydroperoxide wie tert.-Butylhydroperoxid oder Cumolhydroperoxid. Verbindungen dieser Art sind z.B. in „Ullmanns Encyclopädie der Technischen Chemie", 4. Aufl., Bd. 10, S. 563 bis 567, genannt. Der Sauerstoff und die Sauerstoff übertragende Verbindung können auch in Gegenwart von Katalysatoren verwendet werden. Die Umsetzung kann auch, insbesondere bei Verwendung von Persäuren, ohne Zugabe von Carbonsäuren der Formel III durchgeführt werden.

Das neue Verfahren wird z.B. so durchgeführt, dass man pro Mol 1,3-Dien II 1 bis 80, insbesondere 1,5 bis 60 mol der Carbonsäure III und 0,5 bis 10, insbesondere 1 bis 1,5 mol Sauerstoff oder der Sauerstoff übertragenden Verbindung anwendet. Man arbeitet zweckmässig bei Temperaturen zwischen 0 und 200, insbesondere bei 20 bis 120° C. Man wendet z.B. Sauerstoffdrucke von 1 bis 100, insbesondere 1 bis 20 bar, an.

Das Verfahren kann chargenweise oder kontinuierlich, drucklos oder unter Druck durchgeführt werden. Unumgesetzte 1,3-Diene II können gegebenenfalls nach der Umsetzung destillativ von den entstandenen 2-Alkyl-4,4-diacyloxy-2-butenalen I abgetrennt und erneut für die erfindungsgemässe Umsetzung verwendet werden.

Gewöhnlich arbeitet man entweder in einem Überschuss der Carbonsäure III als Lösungsmittel oder in Gegenwart von unter den Reaktionsbedingungen inerten Lösungsmitteln. Als Lösungsmittel dieser Art kommen z.B. Carbonsäureester, wie Essigsäuremethylester, chlorierte Kohlenwasserstoffe, wie Methylenchlorid, Chloroform, Tetrachlorkohlenstoff oder 1,2-Dichlorethan, Kohlenwasserstoffe, wie Alkane, Benzol und alkylierte Benzole, Ether, wie Diethylether, Tetrahydrofuran oder Dioxan in Betracht. Pro Mol Ausgangsverbindung II verwendet man zweckmässigerweise 0,1 bis 80, insbesondere 2 bis 60 mol, des unter den Reaktionsbedingungen inerten Lösungsmittels.

Die Umsetzung kann man bei diskontinuierlicher Arbeitsweise z.B. wie folgt vornehmen: Einer Lösung des 1,3-Diens II in der Carbonsäure III, die gegebenenfalls noch das Lösungsmittel enthält, werden bei der Reaktionstemperatur und dem Reaktionsdruck Sauerstoff oder die Sauerstoff übertragende Verbindung zugeführt. Nach beendeter Zugabe wird gegebenenfalls noch nachgerührt. Durch das auf Raumtemperatur abgekühlte Reaktionsgemisch wird gegebenenfalls Stickstoff geleitet. Anschliessend wird nach dem Abdestillieren des Lösungsmittels und/oder der Carbonsäure III fraktioniert destilliert. Dabei werden gegebenenfalls nicht umgesetzte Ausgangsverbindungen von den gewünschten Aldehyden abgetrennt.

Die nach dem erfindungsgemässen Verfahren mit hoher Selektivität zugänglichen 2-Alkyl-4,4-diacyloxy-2-butenale I stellen wertvolle Zwischenprodukte für die Herstellung von Terpenen wie Retinal, β-Carotin (DE-OS Nr. 2357810) und Apocarotinoiden dar.

Gegenüber den oben genannten bekannten Verfahren, bei denen man andere Ausgangsstoffe oxidiert, zeichnet sich das erfindungsgemässe Verfahren durch den grossen Vorteil aus, dass man anstelle von teuren und/oder toxischen Oxidationsmitteln, wie Selendioxid, Chromsäure, Ozon, Nitrosylchlorid oder Salpetrigsäureestern hier Sauerstoff oder Sauerstoff übertragende Verbindungen, wie Wasserstoffperoxid, Percarbonsäuren oder organische Hydroperoxide verwenden kann.

Das Verfahren der Erfindung ist auch überraschend, denn es war nicht voraussehbar, dass unter den Reaktionsbedingungen 2-Alkyl-4,4-diacyloxy-2-butenale und hierbei ganz überwiegend die Transisomeren entstehen würden. Aus der EP-PS Nr. 5452 ist nämlich bekannt, dass bei der Umsetzung von 2-Alkyl-1,4-diacyloxy-1,3-butadienen mit Carbonsäuren und Sauerstoff in Gegenwart von Palladium oder Platin enthaltenden Katalysatoren 2-Alkyl-1,1,4,4-tetraacyloxy-2-butene gebildet werden.

*Beispiel 1:*

Man gibt zu einer Lösung von 46 g 2-Methyl-1,4-diacetoxy-1,3-butadien in 420 g Eisessig bei 95 ± 2° C unter Rühren innerhalb von 1 h 200 g einer Eisessiglösung, die 11 Gew.-% Peressigsäure enthält. Man rührt weitere 20 min bei dieser Temperatur nach. Unumgesetzte Peressigsäure ist nicht mehr im Reaktionsgemisch nachweisbar. Man zieht nun die Essigsäure am Rotationsverdampfer bei einem Druck von etwa 20 mbar weitgehend ab. Durch fraktionierte Destillation des Rückstands erhält man 38,1 g 2-Methyl-4,4-diacetoxy-2-butenal vom Siedepunkt 94 bis 96°C/0,6 mbar, $n_D^{20}$ = 1,4562 (76% bezogen auf eingesetztes 2-Methyl-1,4-diacetoxy-1,3-butadien).

*Beispiel 2:*

Es wird ein Reaktor mit Flügelrührer (1000 tr/min) verwendet, dessen freies Volumen 0,4 l beträgt. In den Reaktor gibt man pro Stunde bei einer

Innentemperatur von 95 ± 2° C 240 g Eisessig, der 5 Gew.-% 2-Methyl-1,4-diacetoxy-1,3-butadien enthält, und 4Nl Sauerstoff. Der Druck im Reaktor wird durch Aufpressen von Sauerstoff auf 10 bar gehalten. Innerhalb von 96 h werden 1157 g 2-Methyl-1,4-diacetoxy-1,3-butadien zugeführt. Man erhält 23,6 kg Reaktionsaustrag, aus dem nach weitgehendem Abziehen des Eisessigs am Rotationsverdampfer bei rund 20 mbar und fraktionierter Destillation über eine mit Glasringen gefüllte Kolonne (Länge 50 cm, Durchmesser 3 cm) 760 g 2-Methyl-4,4-diacetoxy-2-butenal vom Siedepunkt 90 bis 92° C/0,5 mbar, $n_D^{20} = 1,4578$ (60%, bezogen auf eingesetztes 2-Methyl-1,4-diacetoxy-1,3-butadien) erhalten werden.

Ausgangsprodukt ist laut $^1H$-NMR-Spektrum ($CDCl_3$) im Destillat nicht mehr vorhanden. Es besteht aus dem Transisomeren, das höchstens 10% Cis-2-methyl-4,4-diacetoxy-2-butenal enthält.

*Beispiel 3:*

Man gibt zu einer Lösung von 18,4 g 2-Methyl-1,4-diacetoxy-1,3-butadien in 200 g Eisessig bei 28 ± 2° C innerhalb von 15 min 6,8 g Wasserstoffperoxid (50 Gew.-%ig). Das Reaktionsgemisch wird auf 95 ± 2° C erhitzt und 2 h bei dieser Temperatur gerührt. Nach Abziehen des Eisessigs am Rotationsverdampfer bei einer Badtemperatur von 50° C und 25 mbar erhält man durch fraktionierte Destillation 11 g 2-Methyl-4,4-diacetoxy-2-butenal vom Siedepunkt 90 bis 93° C/0,6 mbar, $n_D^{20} = 1,4598$ (55%, bezogen auf eingesetztes 2-Methyl-1,4-diacetoxy-1,3-butadien).

*Beispiel 4:*

Man gibt zu einer Lösung von 36,8 g 2-Methyl-1,4-diacetoxy-1,3-butadien in 300 g Eisessig unter Rühren bei Raumtemperatur 21,3 g tert.-Butylhydroperoxid (84,4 Gew.-%ig) in 180 g Eisessig. Dann wird auf 95° C aufgeheizt und 4,5 h bei dieser Temperatur gerührt. Nach dem Abziehen des Eisessigs am Rotationsverdampfer erhält man durch fraktionierte Destillation des Rückstands 18,7 g Destillat vom Siedepunkt 92 bis 100° C/1 mbar, $n_D^{20} = 1,4802$, das laut $^1H$-NMR-Spektrum ($CDCl_3$) zu rund 60 Gew.-% aus 2-Methyl-4,4-diacetoxy-2-butenal und zu rund 40 Gew.-% aus unumgesetztem 2-Methyl-1,4-diacetoxy-1,3-butadien besteht.

## Patentansprüche

1. Verfahren zur Herstellung von 2-Alkyl-4,4-diacyloxy-2-butenalen der Formel:

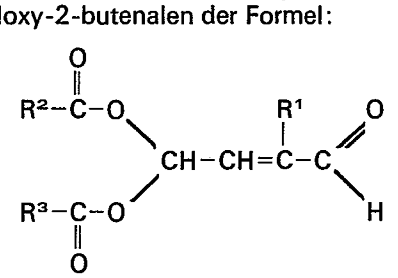

(I)

in der $R^1$ einen Alkylrest mit 1 bis 5 Kohlenstoffatomen und $R^2$ und $R^3$ ein Wasserstoffatom, einen aliphatischen Rest mit 1 bis 15 Kohlenstoffatomen, einen cycloaliphatischen Rest mit 5 bis 7 Kohlenstoffatomen oder einen aromatischen Rest bedeuten, dadurch gekennzeichnet, dass man 2-Alkyl-1,4-diacyloxy-1,3-butadiene der Formel:

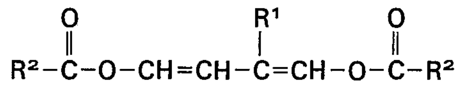

(II)

in der $R^1$ und $R^2$ die obengenannte Bedeutung besitzen, mit Sauerstoff oder Sauerstoff übertragenden Verbindungen und in Gegenwart von Carbonsäuren der Formel:

$R^3-COOH$ (III)

in der $R^3$ die oben genannte Bedeutung besitzt, umsetzt.

2. Verfahren nach Anspruch 1, dadurch gekennzeichnet, dass man pro Mol Dien der Formel (II) 1 bis 80 mol der Carbonsäure der Formel (III) und 0,5 bis 10 mol Sauerstoff oder der Sauerstoff übertragenden Verbindung anwendet.

3. Verfahren nach Anspruch 1, dadurch gekennzeichnet, dass man die Umsetzung bei Temperaturen zwischen 0 und 200° C durchführt.

## Claims

1. A process for the preparation of a 2-alkyl-4,4-diacyloxybut-2-enal of the formula:

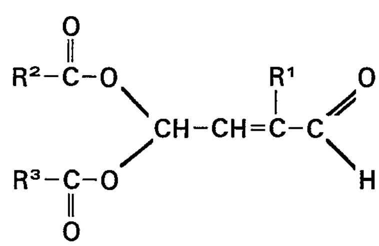

(I)

where $R^1$ is alkyl of 1 to 5 carbon atoms and $R^2$ and $R^3$ are each hydrogen, an aliphatic radical of 1 to 15 carbon atoms, a cycloaliphatic radical of 5 to 7 carbon atoms or an aromatic radical, wherein a 2-alkyl-1,4-diacyloxybuta-1,3-diene of the formula:

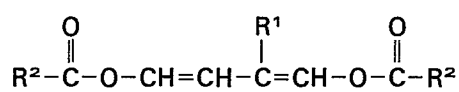

(II)

where $R^1$ and $R^2$ have the above meanings, is reacted with oxygen or an oxygen donor, in the presence of a carboxylic acid of the formula:

$R^3-COOH$ (III)

where $R^3$ has the above meanings.

2. A process as claimed in Claim 1, wherein from 1 to 80 mol of the carboxylic acid of the Formula (III) and from 0.5 to 10 mol of oxygen or of the oxygen donor are employed per mole of the diene of the Formula (II).

3. A process as claimed in Claim 1, wherein the reaction is carried out at from 0 to 200° C.

## Revendications

1. Procédé de préparation de 1-alkyl-4,4-diacyloxy-2-buténals de formule:

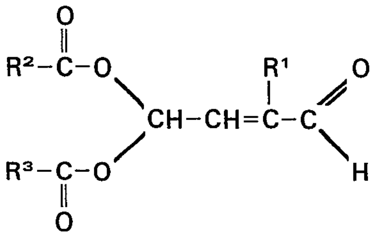

(I)

dans laquelle $R^1$ représente un groupe alkyle en $C_1$-$C_5$ et $R^2$ et $R^3$ représentent un atome d'hydrogène, un groupe aliphatique en $C_1$-$C_{15}$, un groupe cycloaliphatique en $C_5$-$C_7$ ou un groupe aromatique, caractérisé en ce que l'on fait réagir des 2-alkyl-1,4-diacyloxy-1,3-butadiènes de formule:

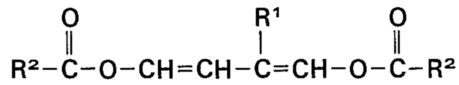

(II)

dans laquelle $R^1$ et $R^2$ ont les significations indiquées ci-dessus, avec l'oxygène ou des composés de l'oxygène et en présence d'acides carboxyliques de formule:

$$R^3-COOH \quad (III)$$

dans laquelle $R^3$ a les significations indiquées ci-dessus.

2. Procédé selon la revendication 1, caractérisé en ce que, pour 1 mol du diène de formule (II), on utilise 1 à 80 mol de l'acide carboxylique de formule (III) et 0,5 à 10 mol d'oxygène ou de composés cédant de l'oxygène.

3. Procédé selon la revendication 1, caractérisé en ce que l'on effectue la réaction à des températures de 0 à 200° C.